(19) Europäisches Patentamt
European Patent Office
Office européen des brevets

(11) Publication number: 0 145 294
B1

(12) **EUROPEAN PATENT SPECIFICATION**

(45) Date of publication of patent specification: **18.10.89**

(51) Int. Cl.$^4$: **C 07 D 249/08,**
A 01 N 43/653

(21) Application number: **84307786.8**

(22) Date of filing: **09.11.84**

(54) **Substituted triazoles, processes for making them, their use as fungicides and fungicidal compositions containing them.**

(30) Priority: **10.11.83 US 551732**

(43) Date of publication of application:
**19.06.85 Bulletin 85/25**

(45) Publication of the grant of the patent:
**18.10.89 Bulletin 89/42**

(84) Designated Contracting States:
**AT BE CH DE FR GB IT LI LU NL SE**

(56) References cited:
**EP-A-0 052 424**
**FR-A-2 300 081**
**FR-A-2 391 200**

**CHEMICAL ABSTRACTS, vol. 101, no. 23, 3rd December 1984, page 652, no. 211152t, Columbus, Ohio, US; & JP - A - 59 104 367 (TAKEDA CHEMICAL INDUSTRIES LTD) 16-06-1984**

**The file contains technical information submitted after the application was filed and not included in this specification**

(73) Proprietor: **ROHM AND HAAS COMPANY**
**Independence Mall West**
**Philadelphia Pennsylvania 19105 (US)**

(72) Inventor: **Fujimoto, Ted Tsutomu**
**44 East Norton Drive**
**Churchville, Pa. 18966 (US)**

(74) Representative: **Angell, David Whilton et al**
**ROHM AND HAAS (UK) LTD. European Operations Patent Department Lennig House 2 Mason's Avenue**
**Croydon CR9 3NB (GB)**

(56) References cited:
**CHEMICAL ABSTRACTS, vol. 98, no. 25, 20th June 1983, page 556, no. 215541m, Columbus, Ohio, US; H. MITSUDERA et al.: "Synthesis and fungicidal activity of 1,2,4-triazole derivatives", & TAKEDA KENKYUSHOHO 1982, 41(3/4), 148-53**

**EP 0 145 294 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention concerns novel substituted triazoles, processes for making them, their use as fungicides and fungicidal compositions containing them.

U.S. Patent No. 4,366,165, EP—A—0052424, FR—A—2300081, JP—A—59104367 and Mitsudera and Konishi [J. Takeda Res. Lab., 41, 148—153 (1982) and Chem Abs. 98, No 25, 215541M] disclose α-(halo-substituted phenyl)-α-alkyl-β-(1,2,4-triazol-1-yl)propanenitriles and their use in combating phytopathogenic fungi. However these references fail to point to the specific class of compounds of the present invention which have a particularly high degree of fungicidal acticity against certain fungi, notably those on wheat and barley.

The present invention provides α-(halo-substituted phenyl)-α-alkyl-β-(1,2,4-triazol-1-yl)propanenitriles characterized in that said propanenitriles are of Formula I below

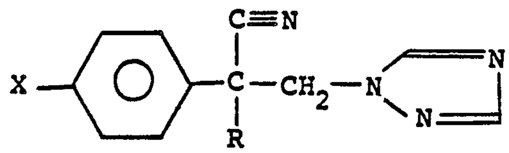 (I)

wherein X is fluoro, chloro or bromo and R is ($C_3$—$C_8$)alkyl with the proviso that when R is branched chain ($C_3$—$C_6$)alkyl the branching does not occur at the α-carbon of the R substituent.

The invention also provides agronomically acceptable acid addition salts and agronomically acceptable metal salt complexes of compounds of Formula I.

The term "alkyl" used herein includes both branched and straight chained alkyl groups but where a specific alkyl group containing no prefix is referred to the n-alkyl group in question is meant, e.g. "butyl" means "n-butyl". Typical alkyl groups are propyl, n-butyl, iso-butyl, pentyl, neo-pentyl, iso-pentyl, hexyl, heptyl, octyl and iso-octyl.

An illustrative but preferred compound of Formula I has the formula

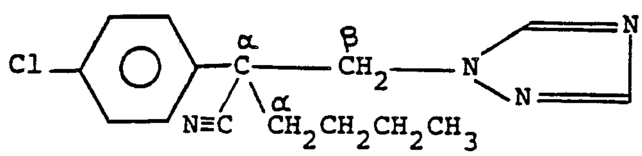

This compound is named herein α-butyl-α-(4-chlorophenyl)-β-(1,2,4-triazol-1-yl)propanenitrile but it could alternatively be named α-butyl-α-(4-chlorophenyl)-1H-1,2,4-triazole-1-propanenitrile.

The acids which can be utilized in making the acid addition salts include, for example, hydrochloric, hydrobromic, nitric, sulfuric, phosphoric, hydroiodic, hydrofluoric, perchloric, p-toluenesulfonic, methanesulfonic, acetic, citric, tartaric, malic, maleic, oxalic, fumaric and phthalic acids.

Preferred metal salt complexes are of the Formula (II):

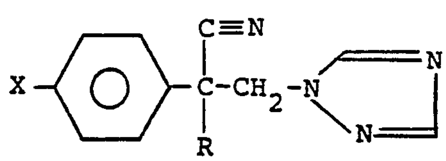 · MX (II)

wherein X and R are as defined in Formula (I) above and M is a cation selected from Group IIA, IB, IIB, VIB, VIIB and VIII of the Periodic Table as shown in Handbook of Chemistry and Physics and X is an anion selected so that the sum of the valence charges of the cation M and anion X equal zero. Typical cations are magnesium, manganese, copper, nickel, zinc, iron, cobalt, calcium, tin, cadmium, mercury, chromium, lead and barium. Typical anions are chloride, bromide, iodide, fluoride, sulfate, bisulfate, perchlorate, nitrate, nitrite, phosphate, carbonate, bicarbonate, acetate, citrate, oxalate, tartrate, malate, maleate, fumarate, p-toluenesulfonate, methanesulfonate, mono- or di-($C_1$—$C_4$)alkyldithiocarbamate and ($C_1$—$C_4$)alkylene-bisdithiocarbamate.

Preferred compounds are those wherein in Formula I X is fluoro or chloro. It is also preferred that R is ($C_3$—$C_6$)alkyl. More preferred compounds are those where X is fluoro or chloro and R is a propyl, butyl, isobutyl or pentyl. The most preferred two compounds are those where X is chloro and R is n- or iso-butyl.

Typical compounds provided by the present invention include:

alpha-(4-chlorophenyl)-alpha-propyl-beta-(1,2,4-triazol-1-yl)propanenitrile

alpha-(4-fluorophenyl)-alpha-propyl-beta-(1,2,4-triazol-1-yl)propanenitrile
alpha-butyl-alpha-(4-chlorophenyl)-beta-(1,2,4-triazol-1-yl)propanenitrile
alpha-butyl-alpha-(4-bromophenyl)-beta-(1,2,4-triazol-1-yl)propanenitrile
alpha-butyl-alpha-(4-fluorophenyl)-beta-(1,2,4-triazol-1-yl)propanenitrile
alpha-(4-chlorophenyl)-alpha-iso-butyl-beta-(1,2,4-triazol-1-yl)propanenitrile
alpha-(4-chlorophenyl)-alpha-pentyl-beta-(1,2,4-triazol-1-yl)propanenitrile
alpha-(4-chlorophenyl)-alpha-isopentyl-beta-(1,2,4-triazol-1-yl)propanenitrile
alpha-(4-fluorophenyl)-alpha-hexyl-beta-(1,2,4-triazol-1-yl)propanenitrile
alpha-(4-fluorophenyl)-alpha-heptyl-beta-(1,2,4-triazol-1-yl)propanenitrile
alpha-(4-chlorophenyl)-alpha-octyl-beta-(1,2,4-triazol-1-yl)propanenitrile
and the agronomically acceptable acid addition salts and metal salt complexes thereof.

The compounds provided by the present invention possess curative, residual and preventive antifungal properties against a broad spectrum of phytopathogenic fungi. They additionally may act as systemic and/or contact fungicides. Examples of such fungi include wheat and barley powdery mildew (*Erysiphe graminis*), rice blast (*Biricularia oryzae*), peanut early leaf spot (*Cercospora arachidicola*), banana Sigatoka (*Mycosphaerella fijiensis*), wheat leaf rust (*Puccinia recondita*), wheat stem rust (*Puccinia graminis*), Septoriosis of wheat (*Septoria* spp.), barley net blotch (*Helminthosporium teres*), grape powdery mildew (*Uncinula necator*), grape black rot (*Guignardia bidwellii*), apple scab (*Venturia inequalis*), apple powdery mildew (*Podosphaera lencotricha*), cucumber powdery mildew (*Sphaerotheca fuliginea*), brown rot of stone fruits (*Monilinia fructicola*) and rice sheath blight (*Rhizoctonia solani*).

The triazoles concerned in the present invention can be prepared by conventional synthesis routes involving the reaction of 1-H-1,2,4-triazole with one or more reagents whereby in one or more steps there is introduced into the 1-position of the triazole a substituent of the formula:

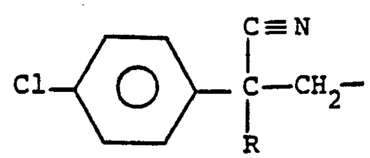

For example, the triazoles may be prepared by nucleophilic displacement of the alkylated phenylacetonitrile bromide (V) by a salt, preferably an alkali metal salt, of the triazole, generally about 1 to about 3 equivalents. This reaction can be run either neat or, preferably, in an appropriate solvent such as dimethylsulfoxide (DMSO), n-dimethyl-formamide, toluene or xylene at a temperature from about 0°C to about 150°C, preferably from about 25 to about 100°C. The bromide (V) is prepared by bromomethylation of the alkylated phenylacetonitrile (IV) by methylenebromide (generally about 1.1 to about 2 equivalents) under basic conditions, e.g., sodium or potassium hydroxide, sodium or potassium hydride, potassium methoxide and potassium-t-butoxide (generally about 1.1 to about 2 equivalents) preferably with the use of a solvent such as DMSO, at a temperature from about 0 to about 150°C, preferably from about 25 to about 100°C. The alkylated phenylacetonitriles (IV) can be prepared by phase transfer alkylation of the appropriately substituted benzylcyanides (III) with generally about 1 to about 2 equivalents of an alkyl halide (RX wherein R is as defined above and X is, for example, Cl, Br, I, tosylate or mesylate) in the presence of a strong base, e.g., 50% (w/w) sodium hydroxide or another metal alkoxide, and a catalyst, e.g., tetrabutylammonium bromide. Both the benzylcyanides and the alkyl halides can be readily prepared by techniques known from the literature. This synthesis scheme is shown below:

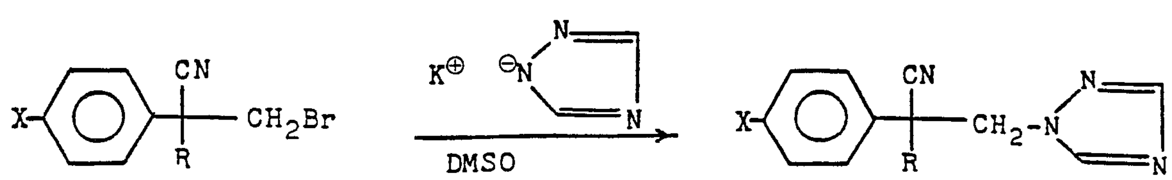


(V) (I)

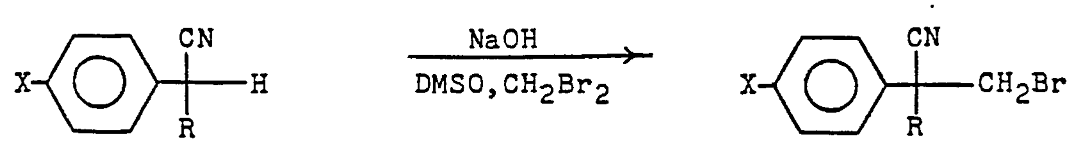


(IV) (V)

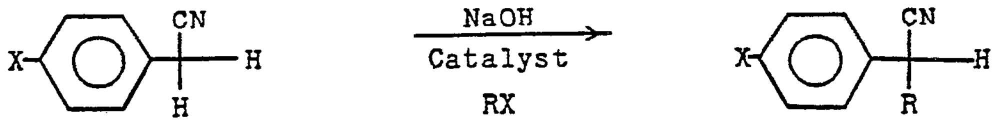


(III)

(IV)

The acid addition salts of the triazoles can be prepared by standard techniques well-known in the art. For example, the triazole of formula (I) can be dissolved in an appropriate solvent such as diethyl ether, tetrahydrofuran, ethanol on methanol or combinations thereof and treated with an equivalent or excess amount of a mineral or organic acid which may or may not be dissolved in an appropriate solvent, the mixture is then either cooled or evaporated to give the salt which can either be used as such or recrystallized from an appropriate solvent or combination of appropriate solvents.

The metal salt complexes of the triazoles of formula I can be prepared by adding dropwise, with stirring, a stoichiometric amount of a metal salt dissolved in an appropriate solvent or combination of solvents to a solution of the triazole of formula (I) dissolved in a similarly appropriate solvent or combination of solvents. The reaction mixture is briefly stirred and the solvent is removed under reduced pressure to give the metal salt complex of the respective triazole.

The metal salt complexes can also be prepared by mixing stoichiometric or excess amounts of the metal salt and a triazole of formula (I) in the desired amount of solvent containing the appropriate adjuvants just prior to spraying the plants. Adjuvants that may be includced in this "in situ" preparation may be detergents, emulsifiers, wetting agents, spreading agents, dispersing agents, stickers or adhesives, which are used in agricultural applications.

Solvents that can be utilized in these procedures include any polar solvent, e.g., water, methanol, ethanol, isopropanol or ethylene glycol and any aprotic dipolar solvent, e.g., dimethylsulfoxide, acetonitrile, dimethylformamide, nitromethane or acetone.

The metal salt cations that can be used in these procedures can be selected from the group consisting of calcium, magnesium, manganese, copper, nickel, zinc, iron, cobalt, tin, cadmium, mercury, chromium, lead and barium. Any appropriate anion, e.g., chloride, bromide, iodide, sulfate, bisulfate, phosphate, nitrate, perchlorate, carbonate, bicarbonate, hydrosulfide, hydroxide, acetate, oxalate, malate or citrate may be utilized as the counterion in the metal salt.

The compounds of formula I possess an asymmetric carbon atom and thus exist as racemic mixtures. The d and l enantiomorphs in these racemic mixtures can be separated via standard techniques such as fractional crystallization with d-tartaric acid, l-tartaric acid or l-quinic acid followed by basification and extraction of the d or l enantiomorph free base. The invention, of course, includes all enantiomorphic forms.

The compounds of formula I, acid addition salts and metal salt complexes provided by the present invention are useful as agricultural fungicides and as such can be applied to various loci such as the seed, the soil (or other growth medium), the foliage or plant habitat. For such purposes one or more of these compounds can be used in the technical or pure form as prepared or as formulations which contain the active ingredient in amounts up to 99% by weight but more usually up to 95%, eg 5—95%, by weight. The compounds are usually used in conjunction with an agronomically acceptable carrier or diluent. Formulations can take the form of wettable powders, emulsifiable concentrates, dusts, granular formulations, aerosols, or flowable emulsion concentrates. In such formulations, the compounds are extended with a liquid or solid carrier or diluent and, when desired, suitable surfactants are incorporated.

It is usually desirable, particularly in the case of foliar spray formulations, to include adjuvants, such as wetting agents, spreading agents, dispersing agents, stickers or adhesives in accordance with agricultural practices. Such adjuvants commonly used in the art can be found in the John W. McCutcheon, Inc. publication "Detergents and Emulsifiers, Annual".

In general, the compounds provided by this invention can be dissolved in certain solvents such as acetone, methanol, ethanol, dimethylformamide or dimethyl sulfoxide and such solutions can be extended with water. The concentrations of the solution can vary from about 1% to about 90% by weight with a preferred range being from about 5% to about 50% by weight.

For the preparation of emulsifiable concentrates, the compound can be dissolved in suitable organic solvents, or a mixture of solvents, together with an emulsifying agent which permits dispersion of the fungicide in water. The concentration of the active ingredient in emulsifiable concentrates is usually from about 10% to about 90% by weight and in flowable emulsion concentrates, this can be as high as about 75% by weight.

Wettable powders suitable for spraying, can be prepared by admixing the compound with a finely divided solid, such as clays, inorganic silicates and carbonates, and silicas and incorporating wetting agents, sticking agents, and/or dispersing agents in such mixtures. The concentration of active ingredients in such formulations is usually in the range of from about 20% to about 98%, preferably from about 40% to about 75%, by weight. A typical wettable powder is made by blending 50 parts of alpha-(4-chlorophenyl)-alpha-butyl-beta-(1,2,4-triazol-1-yl)propanenitrile, 45 parts of a synthetic precipitated hydrated silicon dioxide sold under the trademark Hi-Sil, and 5 parts of sodium lignosulfonate. In another preparation a kaolin type (barden) clay is used in place of the Hi-Sil in the above wettable powder, and in another such preparation 25% by weight of the Hi-Sil is replaced with a synthetic sodium silico aluminate sold under the trademark Zeolex 7.

Dusts are prepared by mixing the active triazole ingredient with finely divided inert solids which can be organic or inorganic in nature. Materials useful for this purpose include botanical flours, silicas, silicates, carbonates and clays. One convenient method of preparing a dust is to dilute a wettable powder with a finely divided carrier. Dust concentrates containing from about 20% to about 80% by weight of the active ingredient are commonly made and are subsequently diluted to from about 1% to about 10% by weight use concentration.

The compounds provided by the invention can be applied as fungicidal sprays by methods commonly employed, such as conventional high-gallonage hydraulic sprays, low-gallonage sprays, air-blast spray, aerial sprays and dusts. The dilution and rate of application can be readily determined by one in the art depending upon the type of equipment used, the desired method, timing and frequency of applications, plants to be treated and diseases to be controlled. Generally, however, the fungicidal compounds provided by the present invention will be applied in an amount of from about 0.1 to about 22 kg of active ingredient per hectare when applied foliarly or to the soil.

As a seed protectant, the amount of the compound coated on the seeds is usually from about 1.4 g to about 113 g of active ingredient per 45 kg of seed and preferably from 2.8 g to about 28 g per 45 kg of seed. As a soil or other growth medium fungicide the compounds can be incorporated in the growth medium or applied to its surface usually at a rate of from about 0.06 to about 22 kg, preferably from about 0.02 to about 11 kg and more preferably from about 0.1 to about 3 kg of active ingredient per hectare. As a foliar fungicide, the compounds are usually applied to growing plants at a rate of from about 0.01 to about 11 kg, preferably from about 0.02 to about 6 and more preferably from about 0.03 to about 1 kg of active ingredient per hectare.

Fungicides which can be used in combination with the fungicides of this invention include all those listed in U.S. Patent No. 4,366,165.

The compounds of formula I and the acid addition salts and metal salt complexes thereof can be advantageously employed in various ways. Since these compounds possess broad spectrum fungicidal activity, they can be employed as fungicides in turf, fruit orchards, vegetable crops, cereal crops, golf course applications and the storage of cereal grain.

The following Examples 1—4 show the preparation of illustrative but preferred compounds of the invention. Example 5 gives fungicidal efficacy data.

## Example 1

Preparation of alpha-butyl-alpha-(4-chlorophenyl)-beta-(1,2,4-triazol-1-yl)-propanenitrile

A) *1-bromo-2-cyano-2-(4-chlorophenyl)hexane*

In a 300 ml three neck flask fitted with a mechanical stirrer, thermometer and addition funnel was added 20.8 g (0.1 mol) of α-(p-chlorophenyl)hexanenitrile, 34.8 g (0.2 mol) of methylene bromide and 50 ml of DMSO. To the reaction flask was added 24 ml of 50% (w/w) sodium hydroxide, dropwise over a 35 minute period. Upon completion of the reaction, it was quenched by adding 500 ml of water. The aqueous mixture was extracted three times with ether, then the combined ether extracts were washed three times with water and once with brine. The organic phase was dried over sodium sulfate, filtered and the solvent removed by rotary evaporation yielding 22.6 g of a yellow oil.

NMR: 60 mHz 0.8—2.0 δ (m, 9H), 4.0 δ (br s, 2H) and 7.4 δ (s, 2H).

B) *Alpha-butyl-alpha-(4-chlorophenyl)-beta-(1,2,4-triazol-1-yl)propanenitrile*

In a single neck 500 ml flask fitted with a magnetic stirrer and drying tube was added 26.6 g (0.09 mol) of 1-bromo-2-cyano-2-(4-chlorophenyl)hexane, followed by 19.0 g of potassium 1-H-1,2,4-triazole (0.18 mol) and 100 ml of DMSO. The reaction was stirred for about 48 hours at room temperature and then stirred for about 24 hours at 70°C. The reaction was quenched by pouring it into 1500 ml of water. The aqueous mixture was extracted four times with ether and the combined ether extracts were washed twice with water and once with brine. The organic phase was dried over sodium sulfate, concentrated and redissolved in a minimum amount of ether. Hexane was added until the solution was cloudy, and then the flask was placed in the freezer. The crystals which formed were filtered off and dried. The filtrate was concentrated to obtain additional less pure material for a total of 13.0 g.

Elemental Analysis — Theoretical (Found): C: 62.36 (62.55, 62.38); H: 5.94 (6.07, 6.00); N: 19.41 (19.22, 19.35); Cl: 12.28 (11.00, 11.90).

## Example 2

Preparation of alpha-(4-chlorophenyl)-alpha-propyl-beta-(1,2,4-triazol-1-yl)propanenitrile

A) *2-(4-chlorophenyl)pentanenitrile*

In a four neck one liter flask fitted with a reflux condenser, mechanical stirrer and addition funnel was placed 227.4 g (1.5 mol) of 4-chlorophenylacetonitrile, 235.6 g (3.0 mol) of 1-chloropropane and 4.8 g

(0.015 mol) of tetrabutylammonium bromide. 300 g (3.75 mol) of 50% (w/w) NaOH was added dropwise over 30 minutes. The reaction mixture quickly exothermed to 50°C and the reaction flask was placed in a cold water bath until the reaction mixture cooled to 35°C. Thereafter, the reaction mixture was heated for an additional 4 hours at 50°C, then the reaction was quenched by adding 1500 ml of water. The mixture was extracted twice with ether and the organic phase was washed with water and then with 200 ml of 10% (w/w) hydrochloric acid. The ether was dried, filtered, concentrated and distilled. The distillate was collected, at 105°C at 0.5 mm, and two main fractions of 156.3 g (88% purity) and 88 g (60% purity) were obtained. The higher purity material was used in the bromomethylation of step B below.

NMR: 60 mHz (d-$CHCl_3$): 1.0—2.0 δ (m, 7H), 3.8—3.9 δ (t, 1H) and 7.5 δ (s, 4H).

B) *1-bromo-2-cyano-2-(4-chlorophenyl)-pentane*

In a 2000 ml four neck flask fitted with a reflex condenser, mechanical stirrer, thermometer and addition funnel was placed 305.0 g (1.57 mol) of 2-(4-chlorophenyl)pentanenitrile, 409.4 g (2.36 mol) of dibromomethane and 400 ml of DMSO. 50% (w/w) sodium hdyroxide (251.2 g, 3.00 mol) was added dropwise over 2 hours with the reaction temperature rising to 50°C. A water bath was used to maintain the reaction at about 40°C and after about 4 hours, the reaction was quenched by adding 100 ml of water. After sitting at room temperature for about 16 hours, the mixture was diluted with 1000 ml of ice water, extracted with ether, the combined ether extracts were washed twice with water and then with 10% (w/w) hydrochloric acid and dried over sodium sulfate. The ether was removed by rotary evaporation and the remaining DMSO removed under high vacuum to yield 361.2 g of the bromide derivative.

NMR: 60 mHz (d-$CHCl_3$) 1.0—2.2 δ (m, 7H), 4.0 δ (br s, 2H) and 7.5 δ (s, 4H).

C) *alpha-propyl-alpha-(4-chloro)-beta-(1,2,4-triazol-1-yl)propanenitrile*

In a single neck one liter flask was placed 361.2 g (1.26 mol) of 1-bromo-2-cyano-2-(4-chlorophenyl)-pentane and 300 ml of DMSO. The potassium salt of 1-H-1,2,4-triazole (270.0 g, 2.52 mol) was added and the flask was placed on a rotary evaporator for 1 hour at 75°C to dissolve the triazole salt. The flask was then heated at 90°C for 4 hours, followed by stirring at room temperature overnight (about 16 hours). The reaction was found to be incomplete and reheated to 120°C for about 12 hours followed by stirring it at 90°C for about 36 hours. Upon completion of the reaction, the reaction mixture was cooled to room temperature and diluted with 1500 ml of ice water. The water was extracted with 250 ml of ether; however, since the product crystallized in the ether layer, this layer was then diluted with 1500 ml of ethyl acetate and extracted. The water layer was additionally extracted twice with ethyl acetate and the combined organic extracts were separated into two batches. Each was extracted with water (6 × 150 ml) and once with brine. The dark brown organic phase was dried over sodium sulfate and then filtered. The solution was treated then with activated charcoal and filtered and stripped to give a crystalline cake which was recrystallized from ethyl acetate:ether. The product was kept in the freezer for 16 hours and then filtered and washed with hexane. The filtrate was concentrated, hexane added and then triturated to yield an additional solid which was combined with the first crop to give 166.8 g of product.

M.P.: 108—110°C.

Elemental Analysis — Theoretical (Found): C: 61.20 (61.16); H: 5.50 (5.56); N: 20.39 (20.26); Cl: 12.91 (12.66).

## Example 3

Preparation of alpha-butyl alpha-(4-fluorophenyl)-beta-(1,2,4-triazol-1-yl)propanenitrile

A) *2-(4-fluorophenyl)hexanitrile*

The phase transfer procedure was employed as with 2-(4-chlorophenyl)pentanenitrile, with 400 g (2.96 mol) of p-fluorophenylacetonitrile, 548 g (5.92 mol) of 1-chlorobutane, 592 g (7.4 mol) of 50% (w/w) sodium hydroxide and 9.07 g (0.3 mol) of tetrabutylammonium bromide. The base was added over 2.5 hours with the reaction temperature rising to 35°C. The reaction was then stirred for about 12 hours at 45°C. Upon completion, the reaction was quenched with water and the mixture was extracted and washed as in Example 1 and then distilled to yield 358.8 g of 91% pure product.

NMR data: 60 mHz (d-$CHCl_3$): 0.8—2.0 δ (m, 9H), 3.7—3.8 δ (t, 1H) and 7.4—7.5 δ (br s, 4H).

B) *1-bromo-2-cyano-2-(4-fluorophenyl)hexane*

The bromomethylation procedure of Example 1 was employed using 358.1 g (1.875 mol) of 50% (w/w) sodium hydroxide, 488.9 g (2.81 mol) of dibromomethane and 400 ml of DMSO. Sodium hydroxide (50%, w/w) was added dropwise over about 2 hours with the reaction exotherming to 90°C. The reaction mixture was cooled to 50°C and the mixture was stirred for 11 hours until the reaction was about 90% complete. The reaction was quenched and worked up in the manner described in Example 1A to yield 379.5 g of a 88% pure product. The product was used directly to prepare the triazole adduct as described in step C below.

NMR: 0.8—2.0 δ (m, 9H), 4.0 δ (br s, 2H), and 7.4 δ (s, 4H).

C) *Alpha-butyl-alpha-(4-fluorophenyl)-beta-(1,2,4-triazol-1-yl)propanenitrile*

In a 2 liter four neck flask was placed 379.5 g (1.33 mol) of 1-bromo-2-cyano-2-(4-fluorophenyl)hexane and 300 ml of DMSO to which 156 g (1.46 mol) of potassium 1-H-1,2,4-triazole was added. The reaction

mixture was stirred overnight at 90°C. The product was worked up in the manner described in Example 1B to yield 340.5 g of a semisolid, 83% pure.

NMR data: 60 mHz (d-$CHCl_3$): 1.0—2.2 δ (m, 9H), 4.8 δ (s, 2H), 7.0—7.8 δ (m, 4H), 8.05 δ (s, 1H) and 8.10 δ (s, 1H).

Example 4

Preparation of alpha-(4-fluorophenyl)-alpha-propyl-beta-(1,2,4-triazol-1-yl)propanenitrile

A) *2-(4-fluorophenyl)pentanenitrile*

To 50.0 g (0.37 mol) of 4-fluorophenylacetonitrile, 0.6 g of tetraethyl ammonium bromide, 50 g (0.407 mol) of 1-bromopropane and 50 ml of DMSO was added 35.5 g (0.44 mol) of 50% (w/w) sodium hydroxide at room temperature. After the addition was complete, the reaction was stirred at 50—60°C for 3 hours. The reaction mixture was extracted with ether, the organic phase washed with water and then 10% (w/w) hydrochloric acid. The ether was dried, filtered and distilled to give 23 g of the product (b.p. 105—110°C at 5 mm).

NMR: 60 mHz (d-$CHCl_3$) 0.9—2.2 δ (m, 7H), 3.8—4.0 δ (t, 1H), 7.0—7.6 δ (m, 4H).

B) *1-bromo-2-cyano-2-(4-fluorophenyl)pentane*

The bromomethylation of the previous examples was employed using 23.0 g (0.129 mol) of 2-(4-fluorophenyl)pentanenitrile and 22.5 g (0.129 mol) of dibromoethane in 50 ml of DMSO. To the reaction mixture was added 11.95 g (0.15 mol) of 50% (w/w) sodium hydroxide while stirring at room temperature. The reaction was heated to 60°C for 3 hours, cooled to room temperature and worked up in the manner described in the previous examples, e.g., Example 3B. There was obtained 20.5 g of the bromide derivative which was used directly below in step C.

b.p. 105—110°C at 5 mm.

NMR: 0.7—2.2 δ (m, 7H), 3.9 δ (q, 2H), and 7.0—7.8 δ (m, 4H).

C) *Alpha-(4-fluorophenyl)-alpha-propyl-beta-(1,2,4-triazol-1-yl)propanenitrile*

To a solution of 8.1 g (0.76 mol) of potassium 1-H-1,2,4-triazole and 30 ml of DMSO was added 15 g (0.58 mol) of 1-bromo-2-cyano-2-(4-fluorophenyl)pentane while maintaining the temperature at 50°C. While stirring, the reaction mixture was heated further to 80°C for 1 hour and then heated at 100°C for 24 hours. The product was worked up as described in Example 3C and after concentration and trituration, 1.3 g of a white solid, m.p. 7—76°C, was obtained.

Elemental Analysis — Theoretical (Found): C: 65.09 (64.80); H: 5.86 (5.95); N: 21.69 (20.77); F: 7.36 (7.02).

NMR: 90 mHz (d-$CHCl_3$) 0.9—1.0 δ (t, 3H), 1.2—1.8 δ (m, 2H), 1.9—2.2 δ (t, 2H), 3.7—3.9 δ (q, 2H), 7.0—7.5 δ (m, 4H) and 7.9 δ (br s, 2H).

Following the procedures above-described there may also be prepared compounds of Formula I (and the acid addition salts and metal salt complexes), wherein (a) X is bromo and R is each and every one of propyl, butyl, iso-butyl, pentyl, iso-pentyl, hexyl, iso-hexyl, heptyl, 2-heptyl, octyl or heptyl substituted with methyl in the 3-, 4- or 5-positions, (b) X is chloro and R is each and every one of iso-butyl, pentyl, iso-pentyl, hexyl, iso-hexyl, heptyl, 2-heptyl, octyl and heptyl substituted with methyl in the 3-, 4- or 5-positions and (c) X is fluoro and R is each and every one of the alkyl substituents listed in (b) immediately above.

Example 5

The compounds of Examples 1—4 were tested for their antifungal activity against wheat leaf rust (WLR), wheat powdery mildew (WPM) and barley spot blotch (BSB). The test procedures were as follows:

A) *Wheat Leaf Rust (Puccinia recondita):* Pennoll wheat seedlings about 7 days old were sprayed to runoff with a solution of the test compound comprising 75 parts per million of the test compound suspended in a 2:1:1 by volume mixture of water, acetone and methanol. After drying, the plants were inoculated with a uredial suspension of *P. recondita* (20,000 uredial/ml). The plants were incubated at 22°C in a mist for 24 hours. After seven days of further incubation on a greenhouse bench, the percent disease control was determined by counting uredial pustules and comparing it to the number of pustules on control plants.

B) *Wheat Powdery Mildew (Erysiphe Graminis* f. sp. *tritici):* Pennoll wheat seedings (7—14 days old) were trimmed to provide a uniform plant height and to facilitate uniform inoculation. Twenty-four hours after their trimming, they were sprayed to runoff with a test compound comprising 5 parts per million of the test compound suspended in a 2:1:1 mixture of water, acetone and methanol. Then mildew spores of wheat powdery mildew cultured on wheat seedlings in a controlled temperature room were shaken from the cultured plants onto the Pennoll wheat seedlings. The inoculated seedlings were kept in the controlled temperature room and subirrigated. The percent disease control was rated 8—10 days after inoculation.

C) *Barley Spot Blotch (Helminthosporium sativium):* Pennrad barley seedlings about 7 days old were sprayed to runoff with a test compound comprising 10 parts per million of the test compound suspended in

a 2:1:1 by volume mixture of water, acetone and methanol. After drying, the plants were inoculated with a conidial suspension of *H. sativum* (20,000 conidia/ml). The plants were incubated at 22°C in a mist for 24 hours. After five days of further incubation on a greenhouse bench, the percent disease control was measured by counting lesions and comparing it to the number of lesions on the control plants.

The results of the tests are presented below in Table 1. The disease control ratings utilized in Table 1 are as follows: A = 97—100% disease control, B = 90—96% disease control, C = 70—89% disease control, D = 50—69% disease control and E = 49% or less disease control.

TABLE 1

| | Disease Control Rating | | |
|---|---|---|---|
| Compound | WLR | WPM | BSB |
| 1 | A | A | B |
| 2 | A | A | A |
| 3 | A | B | B |
| 4 | A | A | C |

**Claims for the Contracting States: BE CH DE FR GB IT LI LU NL SE**

1. An α-(halo-substituted phenyl)-α-alkyl-β-(1,2,4-triazol-1-yl)propanenitrile, characterized in that said propanenitrile is of Formula I below

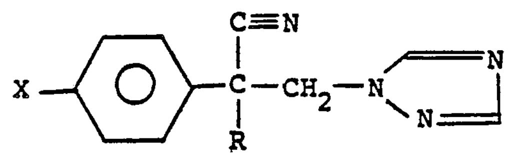

(I)

wherein X is fluoro, chloro or bromo and R is $(C_3—C_8)$alkyl with the proviso that when R is branched chain $(C_3—C_6)$alkyl the branching does not occur at the α-carbon of the R substituent; or an agronomically acceptable acid addition salt or an agronomically acceptable metal salt complex of a propanenitrile of Formula I.

2. A compound as claimed in Claim 1, wherein X is fluoro or chloro.

3. A compound as claimed in Claim 1 or 2, wherein R is $(C_3—C_6)$alkyl.

4. A compound as claimed in Claim 3, wherein R is straight chained alkyl.

5. A compound as claimed in Claims 2 and 3, wherein R is n-propyl, n-butyl, iso-butyl or n-pentyl.

6. A compound as claimed in Claim 3, wherein X is chloro and R is n-propyl or X is fluoro in which case R is n-propyl or n-butyl.

7. A compound as claimed in Claim 3, wherein X is chloro and R is n-butyl.

8. A fungicidal composition containing as active ingredient at least one compound as claimed in any preceding claim and an agronomically acceptable carrier or diluent for the active ingredient.

9. A method for combating phytopathogenic fungi which comprises applying to a plant, to plant seed, to a plant habitat or to growth medium a fungicidally effective amount of a compound as claimed in any one of Claims 1—7.

10. A method as claimed in Claim 9 as applied to combating fungi on wheat.

**Claims for the Contracting State: AT**

1. A fungicidal composition comprising an α-(halo-substituted phenyl)-α-alkyl-β-(1,2,4-triazol-1-yl)propanenitrile of Formula I below

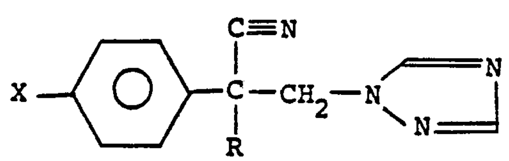

(I)

wherein X is fluoro, chloro or bromo and R is $(C_3—C_8)$alkyl with the proviso that when R is branched chain

($C_3$—$C_6$)alkyl the branching does not occur at the α-carbon of the R substituent; or an agronomically acceptable acid addition salt or an agronomically acceptable metal salt complex of a propanenitrile of Formula I; together with agronomically acceptable diluent or carrier.

2. A composition as claimed in Claim 1, wherein X is fluoro or chloro.

3. A composition as claimed in Claim 1 or 2, wherein R is ($C_3$—$C_6$)alkyl.

4. A composition as claimed in Claim 3, wherein R is straight chained alkyl.

5. A composition as claimed in Claims 2 and 3, wherein R is n-propyl, n-butyl, iso-butyl or n-pentyl.

6. A composition as claimed in Claim 3, wherein X is chloro and R is n-propyl or X is fluoro in which case R is n-propyl or n-butyl.

7. A composition as claimed in Claim 3, wherein X is chloro and R is n-butyl.

8. A method for combating phytopathogenic fungi which comprises applying to a plant, to plant seed, to a plant habitat or to growth medium a fungicidally effective amount of a compound of Formula I as defined in any one of Claims 1—7 optionally in a composition as claimed in any one of Claims 1 to 7.

9. A method as claimed in Claim 8 as applied to combating fungi on wheat.

**Patentansprüche für die Vertragsstaaten: BE CH DE FR GB IT LI LU NL SE**

1. α-(Halogen-subst.-phenyl)-α-alkyl-β-(1,2,4-triazol-1-yl)propannitril, dadurch gekennzeichnet, daß dieses Propannitril der folgenden Formel I entspricht

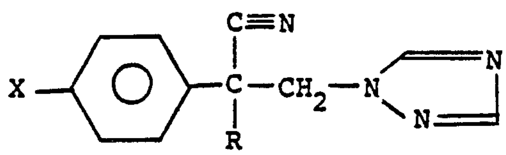

(I)

worin X Fluor, Chlor oder Brom ist und R ($C_3$—$C_8$)-Alkyl ist, unter der Voraussetzung, daß dann, wenn R verzweigtkettiges ($C_3$—$C_6$)-Alkyl ist, die Verzweigung nicht an dem α-Kohlenstoff des R-Substituenten vorliegt, oder ein für landwirtschaftliche Zwecke verträgliches Säureadditionssalz oder ein für landwirtschaftliche Zwecke verträgliches Säureadditionssalz oder ein für landwirtschaftliche Zwecke verträglicher Metallsalzkomplex eines Propannitils der Formel I.

2. Verbindung nach Anspruch 1, worin X Fluor oder Chlor ist.

3. Verbindung nach Anspruch 1 oder 2, worin R ($C_3$—$C_6$)-Alkyl ist.

4. Verbindung nach Anspruch 3, worin R geradkettiges Alkyl ist.

5. Verbindung nach den Ansprüchen 2 und 3, worin R n-Propyl, n-Butyl, Isobutyl oder n-Pentyl ist.

6. Verbindung nach Anspruch 3, worin X Chlor ist und R n-Propyl ist oder X Fluor ist, wobei in diesem Falle R für n-Propyl oder n-Butyl steht.

7. Verbindung nach Anspruch 3, worin X Chlor ist und R n-Butyl ist.

8. Fungizides Mittel, das als Wirkstoff wenigstens eine Verbindung gemäß einem der vorhergehenden Ansprüche und einen für landwirtschaftliche Zwecke verträglichen Träger oder einen für landwirtschaftliche Zwecke verträgliches Verdünnungsmittel für den Wirkstoff enthält.

9. Verfahren zur Bekämpfung von phytopathogenen Pilzen, welches darin besteht, auf eine Pflanze, eine Pflanzensaat, eine Pflanzenwachstumsstelle oder ein Wachstumsmedium einer fungizid wirksamen Menge einer Verbindung gemäß einem der Ansprüche 1 bis 7 aufzubringen.

10. Verfahren nach Anspruch 9, angewendet zur Bekämpfung von Pilzen auf Weizen.

**Patentansprüche für den Vertragsstaat: AT**

1. Fungizides Mittel aus einem α-(Halogen-subst.-phenyl)-α-alkyl-β-(1,2,4-triazol-1-yl)propannitril der folgenden Formel I

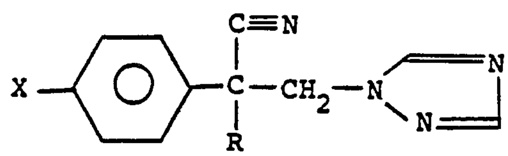

(I)

worin X Fluor, Chlor oder Brom ist und R ($C_3$—$C_8$)-Alkyl ist, unter der Voraussetzung, daß dann, wenn R ein verzweigtkettiges ($C_3$—$C_6$)-Alkyl ist, die Verzweigung nicht an dem α-Kohlenstoff des R-Substituenten auftritt, oder einem für landwirtschaftliche Zwecke verträglichen Säureadditionssalz oder einem für landwirtschaftliche Zwecke verträglichen Metallsalzkomplex eines Propannitrils der Formel I zusammen mit einem für landwirtschaftliche Zwecke verträglichen Verdünnungsmittel oder Träger.

2. Mittel nach Anspruch 1, worin X Fluor oder Chlor ist.

3. Mittel nach Anspruch 1 oder 2, worin R ($C_3$—$C_6$)-Alkyl ist.

4. Mittel nach Anspruch 3, worin R geradkettiges Alkyl ist.

5. Mittel nach den Ansprüchen 2 und 3, worin R n-Propyl, n-Butyl, Isobutyl oder n-Pentyl ist.

6. Mittel nach Anspruch 3, worin X Chlor ist und R n-Propyl ist, oder X Fluor ist, wobei in diesem Falle R n-Propyl oder n-Butyl ist.

7. Mittel nach Anspruch 3, worin X Chlor ist und R n-Butyl ist.

8. Verfahren zur Bekämpfung von phytopatogenen Pilzen, welches darin besteht, auf eine Pflanze, eine Pflanzensaat, eine Pflanzenwachstumsstelle oder ein Wachstumsmedium einer fungizid wirksame Menge einer Verbindung der Formel I, wie in einem der Ansprüche 1 bis 7 definiert, gegebenenfalls in einem Mittel gemäß einem der Ansprüche 1 bis 7, aufzubringen.

9. Verfahren nach Anspruch 8, angewendet zur Bekämpfung von Pilzen auf Weizen.

**Revendications pour les Etats contractants: BE CH DE FR GB IT LI LU NL SE**

1. Un α-(halogénophényl)-α-alcoyl-β-(1,2,4-triazole-1-yl)propanenitrile, ledit propanenitrile étant caractérisé en ce qu'il répond à la formule I ci-dessous

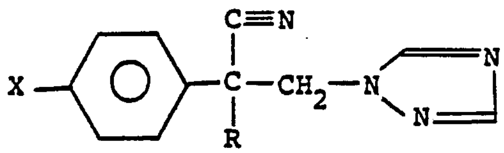

(I)

dans laquelle X est un fluoro, un chloro ou un bromo et R est un alcoyle en $C_3$—$C_8$, sous réserve que, lorsque R est un alcoyle en $C_3$—$C_6$ à chaîne ramifiée, la ramification ne se situe pas sur le carbone α du substituant R; ou un sel d'addition d'acide agronomiquement acceptable ou un sel métallique complexe agronomiquement acceptable d'un propanenitrile de formule I.

2. Un composé selon la revendication 1, où X est un fluoro ou un chloro.

3. Un composé selon la revendication 1 ou 2, où R est un alcoyle en $C_3$—$C_6$.

4. Un composé selon la revendication 3, où R est un alcoyle à chaîne droite.

5. Un composé selon les revendications 2 et 3, où R est un n-propyle, n-butyle, isobutyle ou n-pentyle.

6. Un composé selon la revendication 3, où X est un chloro et R est un n-propyle ou X est un fluoro, auquel cas R est un n-propyle ou un n-butyle.

7. Un composé selon la revendication 3, où X est un chloro et R est un n-butyle.

8. Une composition fongicide contenant comme ingrédient actif au moins un composé selon l'une quelconque des revendications précédentes et un support ou diluant, agronomiquement acceptable, pour l'ingrédient actif.

9. Un procédé de lutte contre les champignons phytopathogènes, qui comprend l'application, à une plante, une graine de plante, un habitat de plante ou un milieu de croissance, d'une quantité fongicide efficace d'un composé selon l'une quelconque des revendications 1 à 7.

10. Un procédé selon la revendication 9 appliqué à la lutte contre les champignons du blé.

**Revendications pour l'Etat contractant: AT**

1. Une composition fongicide comprenant un α-(halogénophényl)-α-alcoyl-β-(1,2,4-triazole-1-yl)propanenitrile de formule I ci-dessous

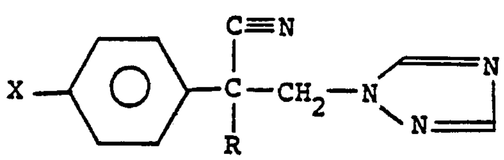

(I)

dans laquelle X est un fluoro, un chloro ou un bromo et R est un alcoyle en $C_3$—$C_8$, sous réserve que, lorsque R est un alcoyle en $C_3$—$C_6$ à chaîne ramifiée, la ramification ne se situe pas sur le carbone α du substituant R; ou un sel d'addition d'acide agronomiquement acceptable ou un sel métallique complexe agronomiquement acceptable d'un propanenitrile de formule I; avec un diluant ou support agronomiquement acceptables.

2. Une composition selon la revendication 1, où X est un fluoro ou un chloro.

3. Une composition selon la revendication 1 ou 2, où R est un alcoyle en $C_3$—$C_6$.

4. Une composition selon la revendication 3, où R est un alcoyle à chaîne droite.

5. Une composition selon les revendications 2 et 3, où R est un n-propyle, n-butyle, isobutyle ou n-pentyle.

6. Une composition selon la revendication 3, où X est un chloro et R est un n-propyle, ou X est un fluoro, auquel cas R est un n-propyle ou un n-butyle.

7. Une composition selon la revendication 3, où X est un chloro et R est un n-butyle.

8. Un procédé de lutte contre les champignons phytopathogènes, qui comprend l'application, à une plante, une graine de plante, un habitat de plante ou un milieu de croissance, d'une quantité fongicide efficace d'un composé de formule I, comme défini dans l'une quelconque des revendications 1 à 7, facultativement dans une composition comme revendiqué dans l'une quelconque des revendications 1 à 7.

9. Un procédé selon la revendication 8 appliqué à la lutte contre les champignons du blé.